# EUROPEAN PATENT SPECIFICATION

(11) **EP 1 693 050 B1**
(45) Date of publication and mention of the grant of the patent: **11.07.2012**
(21) Application number: 06447018.0
(22) Date of filing: 31.01.2006
(51) Int. Cl.: A61K 8/31, A61K 8/63, A61K 8/92, A61Q 19/00, A61Q 5/00

(54) **Care product for skin and/or hair, and the use thereof**
Pflegemittel für Haut und Haare und Verwendung
Composition de soin de la peau et des cheveux, et utilisation

(30) Priority: 02.02.2005 NL 1028164
(43) Date of publication of application: 23.08.2006
(73) Proprietor: Unilever PLC, London EC4Y 0DY (GB); Unilever NV, 3013 AL Rotterdam (NL)
(72) Inventor: Hamidane, Fatima Zahra, 2351 SB Leiderdorp (NL); Lambers, Hans, 2564 LB Den Haag (NL); Landa, Andrew Sjaak, 3461 GP Linschoten (NL)
(74) Representative: Acham, Nicholas Clive

(56) References cited:
- US-A- 3 745 033
- US-A- 4 760 096
- US-A- 6 153 209
- CRODA INC.: SUPER STEROL ESTER, [Online] 17 August 1994 (1994-08-17), XP002386017 Retrieved from the Internet: URL:http://crodausa.com/datasheets/Super_S terol_Ester_DS-63.pdf> [retrieved on 2006-06-14]
- RIT A.W.P. ET AL: 'A NEW BEESWAX DERIVATIVE FOR COSMETIC FORMULATIONS' COSMETICS & TOILETRIES, WHEATON, IL, US vol. 105, no. 8, 01 August 1990, pages 53 - 56,58, XP009016494 ISSN: 0361-4387

## Description

### Technical Field of the Invention

The present invention is directed to a care product, in particular a skin or hair care product. The invention also relates to the use of this product for hair and/or skin care and to the treatment of dry hair and/or dry skin.

### Background of the invention

Our skin is in many ways important and much more than just a covering of our body. The skin is the first line of defence against malicious influences from our environment. The skin prevents overheating, supercooling or dehydration. Moreover is skin able to record and to communicate signals such as pain, heat and cold and pass it on to the central nerve system.

The most important part of the skin acting as a barrier is the horn layer (stratum corneum). This layer is constantly being renewed and, if necessary, repaired from within the epidermis. This happens as skin cells, after being produced continually at the base of the epidermis, gradually move up to the surface. The more the cells approach the surface, the more they transform into horn cells. These horn cells are surrounded by an envelope of skin fats.

In a normal case, vaporization through skin is regulated very precisely. However, when the quantity of skin fat has reduced, water passes through the skin more easily and the skin will dehydrate, causing it to grow red, to flake and crackle.

Washing skin with soap regularly will indeed clean, but also literally 'degrease' it:
skin fats are washed away and the the skin's barrier function is damaged, allowing vapour in the superficial layers of the stratum corneum to vaporize more easily. Frequently washing with water and soap causes dry skin. During winter season there is a high chance for skin dehydration, considering the freezing cold and harsh winds outside, and often a central heating system causing a dry atmosphere inside the house.

A dry skin often occurs with babies and children. During pregnancy, a baby is surrounded by amniotic fluid for nine months. To protect the baby's skin against this fluid, a natural protective coat is provided, called vernix caseosa (vernix), also known as skin grease. This white layer, which grows from the fourth until the last month of the pregnancy, will protect the baby during pregnancy and the first hours after birth. A small residue of vernix will often remain on the baby after birth. Obstetricians and nurses know all too good the special properties of vernix, and leave it on the baby right after birth. The special properties of vernix are protection against amniotic fluid (water repellent) and infections. At the same time, the skin can breathe (pass vapour), and waste products can be removed through this layer.

Because of its protecting properties and its effect on skin maturation, vernix is considered as a promising therapeutical agent. The application of natural vernix on a clinical level is however limited, partly caused by the difficult acquisition of large quantities, the danger of disease transmission and the physical properties of natural vernix, having a different consistency *in utero* than *ex utero* and being not completely soluble in conventional solvents as undiluted ethanol, 95% ethanol, ethanol, iso-propanol or combinations of chloroform and methanol. Applying vernix in a controlled and uniform fashion on a surface is therefore quite difficult.

Literature reports the development of compositions that try to imitate the special properties of vernix. For instance WO03/092646 describes a composition and a method to produce the mentioned composition that simulates the hydrating, cleansing and other properties of vernix. The composition contains hydrated synthetic cells in a lipidic matrix to provide the rheologic properties that are essentially equivalent to those of natural vernix. The composition can also contain proteins. In an embodiment the composition contains cubosomes (Cubic Liquid Crystalline Nanoparticles)/water with a maximum of 30 % proteins and approximately 5 to 30 % lipids. The composition can be used to wash the skin of a newborn baby, providing a hydrating and protecting layer among others.

The present invention aims to provide an improved care product, suited for skin care. In particular the present invention aims to provide a care product having an activity which is similar of that of natural vernix.

### Summary

For this purpose, the present invention provides a care product, particularly suitable for skin and/or hair care, comprising a synthetic beeswax, a C₁₀-C₃₀ cholesterol, a sterol ester, squalane, and C₁₈-C₃₆ triglycerids, wherein the care product comprises the ingredients of maximum 5 wt% (weight percentage), and preferably in a combined quantity ranging from 0.01 to 2 wt%. In another embodiment the invention provides a care product as defined above characterised in that the said sterol ester is a lanosterol ester.

In yet another embodiment the care product according to the invention also contains one or more C₆-C₁₀ triglycerides.

In a further embodiment the invention provides a care product characterised in that the product also comprises tocopherol.

The present invention concerns a care product comprising a composition with the properties similar to those of a newly born baby's skin grease layer, the vernix caseosa, herein simply called vernix. Because of the specific product's composition and because of the use of specific ingredients in particular concentrations and in specific mutual proportions, a care product is obtained that simulates the beneficial effect and the properties of natural vernix caseosa excellently and very efficiently. The present care product has a composition that provides a better simulation and a closer resemblance to natural vernix, compared to compositions currently known.

The present invention obtains its unique vernix-like properties, partly due to specific ingredients applied in particular concentrations and specific mutual proportions in the composition. The present composition is in particular perfectly balanced in order to provide a product capable of adequately protecting and intensively hydrating the skin, while not closing up the skin, so that the skin is allowed to pass vapour. It provides the same characteristics as vernix caseosa: water-repellent, vapour transpiring, wound healing, protection against dehydration and infections and it does not interfere with the removal of skin waste products.

The present care products are particularly suitable for the treating of dry skin and/or hair, from babies and older children as well as from adults. The skin not only remains soft and gentle but is also protected against dehydration and external influences. The skin is protected and intensively hydrated, without being closed up.

The care product provides an intensive protective layer, immediately noticeable after application and allowing vapour to pass through and the skin to breathe and to remove waste products. The care product thus provides a natural protection against the dehydrating effects of water, wind, cold and central heating. It not only prevents dehydration, but has a hydrating effect, keeping up the natural liquid balance. This helps to naturally protect the skin and gives it a soft touch.

In another aspect of the invention a method is provided for the care of skin and/or hair including:
- Bringing said skin and/or hair into contact with a care product according tho the present invention, during an appropriate period of time, and
- Optionally rinsing the said skin after an appropriate contact period.

Some preferred embodiments of a care product for skin and/or hair care are described below, in order to better demonstrate the characteristics of the invention.

### Detailed description of the invention

The invention concerns a skin and/or hair care product, comprising the following elements: synthetic beeswax, C₁₀-C₃₀ cholesterol, a (lano)sterol ester, and squalane. The product additionally comprises C₁₈-C₃₆ triglycerids. The product consists of these ingredients for a combined quantity of maximum 5 wt%, preferably maximum 3 wt%, and ideally for a combined quantity between 0.01 and 2 wt%.

Another embodiment of the invention provides a care product with the characteristic that the product also contains tocopherol.

Yet another embodiment the product according to the invention also comprises one or more C₆-C₁₀ triglycerids, and for example C₈ (caprylic acid) and C₁₀ (caprinic acid).

The care product contains additional ingredients to reach the total mass of 100 wt%, such as and limited to water, glycerin, synthetic odours, colouring agent, preservatives, emulsifying agents, emmolients, stabilizers and similar.

The care product comprises for example (but not limited to) a cream, ointment, lotion, soap, bath milk, bath oil, massage oil, or similar. The care product can possibly be applied in (soaked) tissues, gauzes, cloths or similar. In a particularly desired embodiment the care product includes a cream, a body milk or a bath milk.

Products from the said invention are suitable to be used anywhere on the body and are easily spreadable. They are suitable for daily use with babies, children and adults. The advantageous characteristic of the products is that they leave a noticeable soft layer on the skin, thus providing the skin against external influences, but at the same time allowing the skin to breathe. The product helps the skin to remain healthy and soft. The product has a neutral pH value, does not contain any colour agents, alcohols or soap. The care product from the said invention helps to repair dry spots on the skin, but is equally suited for the treatment of dry hair. The product according to the invention has a softening effect and repairs the normal balance of the skin or hair. The care product can be used economically, is easy to use and gives accurate results relatively quick.

The product contains synthetic beeswax. Beeswax softens but also stabilizes the product and has, as a natural product, a favourable effect on the skin. Beeswax is a complex mixture of elements. By using beeswax in a care product according to the invention, active ingredients can be passed on to the skin during a prolonged period, thus avoiding the use of unwanted high concentrations of active elements. In addition to this, beeswax is a known resource for ω-hydroxy fatty acids, used by skin as materials for ceramids. In that way, beeswax greatly contributes to the skin's hydrology. Beeswax protects the skin without closing it up, gives consistency to the product and has emulsifying properties. Beeswax is a mixture of long-chain (mainly C₂₀-C₃₀) fatty acids, alcohols, and derived esters from it, most of which are encountered naturally in skin. In a particular embodiment the care product from the invention comprises 0.01 to 0.2 wt% synthetic beeswax, and for instance 0.015; 0.030; 0.045; 0.060; 0.075; 0.090; 0.10 or 0.15 wt% synthetic beeswax.

Other ingredients of the care product according to the present invention comprise C₁₀-C₃₀ cholesterol, and a sterol ester (for example lanosterol). The product comprises 0.04 to 0.5 wt% of these ingredients and for example 0.045; 0.09; 0.10; 0.25 or 0.45 wt% of these ingredients. C₁₀-C₃₀ cholesterol regulates the evaporation of fluid through skin. Lanosterol, a predecessor of cholesterol, is a constituent of lanolin, and is being produced on the skin. Lanosterol has the particular characteristic of being able to convert thin running fluids into gel-like substances. This makes the employment of certain source materials more pleasant. Lanosterol therefore enables the combination of both functional as sensory qualities. Cholesterol and (chole)sterol-esters are, alongside ceramids and volatile fatty acids, essential ingredients of the skin's lipid barrier.

Squalane is another ingredient from the said care product. The care product comprises preferably 0.01 to 0.1 wt% squalane and for example 0.01; 0.015; 0.03; 0.045; 0.07; 0.09 or 0.1 wt% squalane. Squalane is an intermediary product in the synthesis of cholesterol. Squalane is encountered naturally in skin, and is capable of simulating the skin's capacities such as regenerating, nourishing, hydrating and protecting itself. It can penetrate the skin, is a natural moisturizer, supples the skin and improves the skin's elasticity. Moreover, squalane has a bactericidal effect when applied in a correct concentrated form. It helps preventing the creation of hyperpigmentation and protects against sun radiation. It is an important oil which prevents skin decay and has the special property of accelerating wound healing. Squalane is related to squalene, which is encountered naturally in skin.

Another embodiment of the care product comprises also 0.1 to 0.5 wt% C₁₈-C₃₆ triglycerids, and for example 0.15; 0.2; 0.3; 0.4 or 0.5 wt% C₁₈-C₃₆ triglycerids. These triglycerids comprise for example stearic acid, oleic acid, linoleic acid, linolenic acid, arachidonic acid, icosanoic acid, eicosapentaenic acid, cetoleinic acid, behenic acid, nervonic acid, docosahexaenoic acid, lignoceric acid, cerotic acid, carboceric acid, octacosanoic acid, melissic acid, lacceroic acid, ceromelissic acid, geddic acid, ceroplastic acid. In brief, C₁₈-C₃₆ triglycerids comprise unsaturated, mono- , di- and polyunsaturated fatty acids.

One other embodiment of the care product may also contain 0.01 to 10.0 wt% C₆₋C₁₀ triglycerids, and for example 0.01; 0.03; 0.50; 0.80; 1.0; 2.0; 3.0; 4.0; 5.0; 6.0; 6.5; 7.0; 7.5; 8.0; 9.0 or 9.5 wt% C₆-C₁₀ triglycerids. These triglycerids contain for example caproic acid, enantic acid, caprylic acid, pelargonic acid or capric acid. in a particularly desired embodiment the care product contains caprylic acid and capric acid.

Yet another embodiment of the invention provides a care product, characterised in that it comprises tocopherol. Tocopherol, also known as Vitamin E, is a particularly effective antioxidant which protects the subcutaneous membranes against oxidation.

One specially desired embodiment of the invention provides a care product, characterised in that the proportion of the C₆-C₁₀ cholesterol and the sterol ester of the synthetic beeswax is 3 to ± 0.75.

Another specially desired embodiment of the invention provides a care product, characterised in that the proportion of the entirety of the ingredients C₁₀-C₃₀ cholesterol, a lanosterol ester and tocopherol to the synthetic beeswax is 3 to ± 0.75.

Another specially desired embodiment of the invention provides a care product, characterised in that the proportion of the C₁₀-C₃₀ cholesterol and the sterol ester to the squalane is 4.5 ± 1.125.

One other specially desired embodiment of the invention provides a care product, characterised in that the proportion of the entirety of the ingredients C₁₀-C₃₀ cholesterol, a lanosterol ester and tocopherol to the squalane is 4.5 to ± 1.125.

Another specially desired embodiment of the invention provides a care product, characterised in that the proportion of the entirety of the ingredients C₁₀-C₃₀ cholesterol and the sterol ester to the C₁₈-C₃₆ triglycerids is 1.5 to ± 0.375.

Another embodiment provides a care product, characterised in that the proportion of the entirety of the ingredients C₁₀-C₃₀ cholesterol, a lanosterol ester and tocopherol to the C₁₈-C₃₆ triglycerids is 1.5 to ± 0.375.

A particularly desired embodiment of the invention provides a care product, characterised in that the proportion of the synthetic beeswax to the squalane is 1.5 to ± 0.375.

Another embodiment provides a care product featuring the proportion of the C₁₈-C₃₆ triglycerids to the synthetic beeswax of 2 to ± 0.5.

Yet another embodiment provides a care product characterised in that the proportion of the C₁₈-C₃₆ triglycerids to the squalane is 3 to ± 0.75.

The care product can be supplied in a concentrated form (as a so called "leaven on" product, for example as an ointment or cream) and in an unconcentrated form (as a so called "rinse off' product, for example as shampoo or bath milk). The concentration of above-mentioned specific ingredients is preferably 5 to 15 % in a concentrated form, and preferably 10 % higher than the concentration of above-mentioned specific ingredients in the unconcentrated form. Another preferred embodiment the concentrated form of the care product only contains C₁₈-C₃₆ triglycerids.

An embodiment of the invention provides a care product characterised in that it comprises, among other things, 0.015 wt% synthetic beeswax, 0.045 wt% of the ingredients C₁₀-C₃₀ cholesterol, a lanosterol ester and tocopherol, and 0.01 wt% squalane. Such a product is particularly suitable for application on skin (or hair) in an unconcentrated form during an appropriate contact period, after which it is rinsed off the application surface.

Another embodiment of the invention provides a care product characterised in that it comprises, among other things, 0.15 wt% synthetic beeswax, 0.45 wt% the ingredients C₁₀-C₃₀ cholesterol, a lanosterol ester and tocopherol, 0.1 wt% squalane and 0.3 wt% C₁₈₋C₃₆ triglycerids. Such a product is particularly suitable to be applied on skin (or hair) in a concentrated form during an appropriate contact period, after which it need not be rinsed off the application surface.

The care product according to the present invention obtains unique qualities that are similar to the healing qualities of natural vernix caseosa, thanks to the use of specific concentrations of individual ingredients and thanks to use of those ingredients in specific mutual relations. In particular it provides protection to the skin (the skin is covered in a layer of the product), and an excellent fluid regulation, while not hindering the vaporising ability of the skin. In spite of the protective layer on the skin, it continues to pass on vapour. The mutual proportions and specific applied quantities of the said ingredients are crucial to the good effect of the care product.

The invention will be illustrated with the following not limiting examples. Tables I, II and III depict three preferred embodiments after the present invention, containing respectively a body milk, a bath milk and a cream. These examples are not limiting in respect to the quantity of the applied ingredients. Within the scope of the invention, the indicated quantities of the ingredients in the product may obviously vary.

**Table I Body milk**

| **Composition** | **Quantity (in wt%)** |
|---|---|
| Synthetic beeswax | From 0.10 to 0.30 % |
| C₁₀-C₃₀ cholesterol / lanosterol esters / tocopherol | From 0.20 to 0.50 % |
| Cholesterol / tocopherol | From 0.10 to 0.30 % |
| C₁₈-C₃₆ triglycerids | From 0.20 to 0.50 % |
| C₆-C₁₀ triglycerids | From 5.0 to 7.5 % |
| Squalane | From 0.01 to 0.30 % |
| Water and other ingredients | Up to 100 % |

**Table II Bath milk**

| **Composition** | **Quantity (in wt%)** |
|---|---|
| Synthetic beeswax | From 0.01 to 0.030 % |
| C₁₀-C₃₀ cholesterol / lanosterol esters / tocopherol | From 0.02 to 0.05 % |
| Cholesterol / tocopherol | From 0.01 to 0.03 % |
| C₆-C₁₀ triglycerids | From 0.01 to 1.0 % |
| Squalane | From 0.01 to 0.05 % |
| Water and other ingredients | Up to 100 % |

**Table III Nutritious cream**

| **Composition** | **Quantity (in wt%)** |
|---|---|
| Synthetic beeswax | From 0.10 to 0.30 % |
| C₁₀-C₃₀ cholesterol / lanosterol esters / tocopherol | From 0.10 to 0.50 % |
| Cholesterol / tocopherol | From 0.10 to 0.30 % |
| C₁₈-C₃₆ triglycerids | From 0.10 to 0.50 % |
| C₆-C₁₀ triglycerids | From 5.0 to 7.5 % |
| Squalane | From 0.10 to 0.30 % |
| Water and other ingredients | Up to 100 % |

## Claims

1. Care product, particularly adapted for skin and/or hair care, comprising water and further comprising the ingredients a synthetic beeswax, a C₁₀-C₃₀ cholesterol, a sterol ester, squalane and C₁₈-C₃₆ triglycerids, and wherein the case product comprises the ingredients of maximum 5 wt%, and preferably as a complete quantity ranging from 0.01 to 2 wt%.

2. Care product according to claim 1, **characterised in that** the product comprises 0.01 to 0.2 wt% synthetic beeswax.

3. Care product according to any of the claims 1 or 2, **characterised in that** the product comprises 0.04 to 0.5 wt% of the ingredients C₁₀-C₃₀ cholesterol and a sterol ester.

4. Care product according to any of the claims 1 to 3, **characterised in that** the product comprises 0.01 to 0.1 wt% squalane.

5. Care product according to any of the claims 1 to 4, **characterised in that** the product comprises 0.1 to 0.5 wt% C₁₈-C₃₆ triglycerids.

6. Care product according to any of the claims 1 to 5, **characterised in that** the proportion of the synthetic beeswax to the squalane is 1.5 to ± 0.375.

7. Care product according to any of the claims 1 to 6, **characterised in that** the proportion of the C₁₀-C₃₀ cholesterol and the sterol ester to the synthetic beeswax is 3 to ± 0.75.

8. Care product according to any of the claims 1 to 7, **characterised in that** the proportion of the C₁₀-C₃₀ cholesterol and the sterol ester to the squalane is 4.5 to ± 1.125.

9. Care product according to any of the claims 1 to 8, **characterised in that** the proportion of the C₁₀-C₃₀ cholesterol and the sterol ester to the C₁₈-C₃₆ triglycerids is 1.5 to ± 0.375.

10. Care product according to any of the claims 1 to 9, **characterised in that** the proportion of the C₁₈-C₃₆ triglycerids to the synthetic beeswax is 1.5 to ± 0.375.

11. Care product according to any of the claims 1 to 10, **characterised in that** the proportion of the C₁₈-C₃₆ triglycerids to the squalane is 1.5 to ± 0.375.

12. Care product according to any of the claims 1 to 11, **characterised in that** the product also comprises tocopherol.

13. Care product according to any of the claims 1 to 8, **characterised in that** the product also comprises C₆-C₁₀ triglycerids.

14. Care product according to any of the claims 1 to 13, **characterised in that** the product comprises additional ingredients, selected from glycerine, synthetic odours, coloring agents, preservatives, emulsifying agents, emmolients, stabilizers and similar, which are present in a complete quantity to 100 wt%.

15. Care product according to any of the claims 1 to 13, **characterised in that** the product comprises a cream, ointment, shampoo, lotion, soap, bath milk, bath oil, massage oil or similar.

16. Non-therapeutic method for skin and/or hair treatment, comprising
- Bringing into contact during an appropriate contact period the said skin and/or hair with the care product, according to one of above mentioned claims 1 to 15, and
- Optionally rinsing the said skin after an appropriate contact period.

## Patentansprüche

1. Pflegeprodukt, das zur Haut- und/oder Haarpflege besonders angepasst ist, das Wasser umfasst und außerdem die Ingredienzien ein synthetisches Bienenwachs, ein C₁₀-C₃₀-Cholesterin, einen Sterolester, Squalan und C₁₈-C₃₆-Triglyceride umfasst und wobei das Pflegeprodukt die Ingredienzien mit maximal 5 Gew.-% und vorzugsweise als eine vollständige Menge im Bereich von 0,01 bis 2 Gew.-% umfasst.

2. Pflegeprodukt gemäß Anspruch 1, **dadurch gekennzeichnet, dass** das Produkt 0,01 bis 0,2 Gew.-% synthetisches Bienenwachs umfasst.

3. Pflegeprodukt gemäß einem der Ansprüche 1 oder 2, **dadurch gekennzeichnet, dass** das Produkt 0,04 bis 0,5 Gew.-% der Ingredienzien C₁₀-C₃₀-Cholesterin und einen Sterolester umfasst.

4. Pflegeprodukt gemäß einem der Ansprüche 1 bis 3, **dadurch gekennzeichnet, dass** das Produkt 0,01 bis 0,1 Gew.-% Squalan umfasst.

5. Pflegeprodukt gemäß einem der Ansprüche 1 bis 4, **dadurch gekennzeichnet, dass** das Produkt 0,1 bis 0,5 Gew.-% C₁₈-C₃₆-Triglyceride umfasst.

6. Pflegeprodukt gemäß einem der Ansprüche 1 bis 5, **dadurch gekennzeichnet, dass** das Verhältnis des synthetischen Bienenwachses zu dem Squalan 1,5 ± 0,375 ist.

7. Pflegeprodukt gemäß einem der Ansprüche 1 bis 6, **dadurch gekennzeichnet, dass** das Verhältnis des C₁₀-C₃₀-Cholesterins und des Sterolesters zu dem synthetischen Bienenwachs 3 ± 0,75 ist.

8. Pflegeprodukt gemäß einem der Ansprüche 1 bis 7, **dadurch gekennzeichnet, dass** das Verhältnis des C₁₀-C₃₀-Cholesterins und des Sterolester zu dem Squalan 4,5 ± 1,125 ist.

9. Pflegeprodukt gemäß einem der Ansprüche 1 bis 8, **dadurch gekennzeichnet, dass** das Verhältnis des C₁₀-C₃₀-Cholesterins und des Sterolesters zu den C₁₈-C₃₆-Triglyceriden 1,5 ± 0,375 ist.

10. Pflegeprodukt gemäß einem der Ansprüche 1 bis 9, **dadurch gekennzeichnet, dass** das Verhältnis der C₁₈-C₃₆-Triglyceride zu dem synthetischen Bienenwachs 1,5 ± 0,375 ist.

11. Pflegeprodukt gemäß einem der Ansprüche 1 bis 10, **dadurch gekennzeichnet, dass** das Verhältnis der C₁₈-C₃₆-Triglyceride zu dem Squalan 1,5 ± 0,375 ist.

12. Pflegeprodukt gemäß einem der Ansprüche 1 bis 11, **dadurch gekennzeichnet, dass** das Produkt auch Tocopherol umfasst.

13. Pflegeprodukt gemäß einem der Ansprüche 1 bis 8, **dadurch gekennzeichnet, dass** das Produkt auch C₆-C₁₀-Triglyceride umfasst.

14. Pflegeprodukt gemäß einem der Ansprüche 1 bis 13, **dadurch gekennzeichnet, dass** das Produkt zusätzliche Ingredienzien, ausgewählt aus Glycerin, synthetischen Geruchsstoffen, Färbemitteln, Konservierungsmitteln, Emulgiermitteln, Emollentien, Stabilisatoren und ähnlichen, umfasst, die in einer vollständigen Menge auf 100 Gew.-% vorliegen.

15. Pflegeprodukt gemäß einem der Ansprüche 1 bis 13, **dadurch gekennzeichnet, dass** das Produkt eine Creme, eine Salbe, ein Shampoo, eine Lotion, eine Seife, eine Bademilch, ein Badeöl, ein Massageöl oder ähnliches umfasst.

16. Nicht-therapeutisches Verfahren zur Haut- und/oder Haarbehandlung, umfassend
- In-Kontakt-Bringen der Haut und/oder des Haars mit dem Pflegeprodukt gemäß einem der oben genannten Ansprüche 1 bis 15 während eines geeigneten Kontaktzeitraums und
- gegebenenfalls Spülen der Haut nach einem geeigneten Kontaktzeitraum.

## Revendications

1. Produit de soins, particulièrement adapté aux soins de la peau et/ou des cheveux, comprenant de l'eau et comprenant, en outre, les ingrédients suivants : une cire d'abeilles synthétique, un cholestérol C₁₀-C₃₀, un ester de stérol, un squalane et des triglycérides C₁₈-C₃₆, et dans lequel le produit de soins comprend ces ingrédients à raison de 5 % en poids maximum, et de préférence, à raison d'une quantité totale allant de 0,01 à 2 % en poids.

2. Produit de soins selon la revendication 1, **caractérisé en ce que** le produit comprend 0,01 à 0,2 % en poids de cire synthétique.

3. Produit de soins selon l'une quelconque des revendications 1 ou 2, **caractérisé en ce que** le produit comprend 0,04 à 0,5 % en poids d'ingrédients représentés par le cholestérol C₁₀-C₃₀ et un ester de stérol.

4. Produit de soins selon l'une quelconque des revendications 1 à 3, **caractérisé en ce que** le produit comprend 0,01 à 0,1 % en poids de squalane.

5. Produit de soins selon l'une quelconque des revendications 1 à 4, **caractérisé en ce que** le produit comprend 0,1 à 0,5 % en poids de triglycérides C₁₈-C₃₆.

6. Produit de soins selon l'une quelconque des revendications 1 à 5, **caractérisé en ce que** la proportion de cire d'abeilles synthétique au squalane est de 1,5 ± 0,375.

7. Produit de soins selon l'une quelconque des revendications 1 à 6, **caractérisé en ce que** la proportion du cholestérol C₁₀-C₃₀ et de l'ester de stérol à la cire d'abeilles synthétique est de 3 ± 0,75.

8. Produit de soins selon l'une quelconque des revendications 1 à 7, **caractérisé en ce que** la proportion du cholestérol C₁₀-C₃₀ et de l'ester de stérol au squalane est de 4,5 ± 1,125.

9. Produit de soins selon l'une quelconque des revendications 1 à 8, **caractérisé en ce que** la proportion du cholestérol C₁₀-C₃₀ et de l'ester de stérol aux triglycérides C₁₈-C₃₆ est de 1,5 ± 0,375.

10. Produit de soins selon l'une quelconque des revendications 1 à 9, **caractérisé en ce que** la proportion des triglycérides C₁₈-C₃₆ à la cire d'abeilles synthétique est de 1,5 ± 0,375.

11. Produit de soins selon l'une quelconque des revendications 1 à 10, **caractérisé en ce que** la proportion des triglycérides C₁₈-C₃₆ au squalane est de 1,5 ± 0,375.

12. Produit de soins selon l'une quelconque des revendications 1 à 11, **caractérisé en ce que** le produit comprend également du tocophérol.

13. Produit de soins selon l'une quelconque des revendications 1 à 8, **caractérisé en ce que** le produit comprend également des triglycérides C₆-C₁₀.

14. Produit de soins selon l'une quelconque des revendications 1 à 13, **caractérisé en ce que** le produit comprend des ingrédients supplémentaires, choisis parmi la glycérine, les odeurs de synthèse, les agents colorants, les conservateurs, les agents émulsifiants, les émollients, les stabilisants et autres ingrédients similaires, qui sont présents en une quantité suffisante pour aller jusqu'à 100 % en poids.

15. Produit de soins selon l'une quelconque des revendications 1 à 13, **caractérisé en ce que** le produit comprend une crème, une pommade, un shampooing, une lotion, un savon, un lait de bain, une huile de bain, une huile de massages ou autres produits similaires.

16. Procédé non thérapeutique pour traiter la peau et/ou les cheveux, comprenant
- la mise en contact pendant une période de contact appropriée de ladite peau et/ou desdits cheveux avec un produit de soins, selon l'une des revendications 1 à 15 mentionnées ci-dessus, et
- éventuellement, le rinçage de ladite peau à l'issue de la période de contact appropriée.
